# DEMANDE DE BREVET EUROPEEN

(11) **EP 1 703 281 A1**
(43) Date de publication de la demande: **20.09.2006**
(21) Numéro de dépôt: 05290579.1
(22) Date de dépôt: 16.03.2005
(51) Int. Cl.: G01N 33/50, C12Q 1/04, C12R 1/24, C12R 1/225

(54) **Souches bactériennes induisant une diminution de l'expression de Rbx1**

(71) Demandeur: COMPAGNIE GERVAIS DANONE, 92302 Levallois-Perret Cedex (FR); INSTITUT PASTEUR, 75724 Paris Cédex 15 (FR)
(72) Inventeur: Pedron, Thierry, 78180 Montigny le Bretonneux (FR); Sansonetti, Philippe, 75014 Paris (FR); Girardin, Stéphane, 94300 Vincennes (FR); Bourdet-Sicard, Raphaelle, 91120 Palaiseau (FR)
(74) Mandataire: Vialle-Presles, Marie José

(57) **Abrégé**

L'invention concerne la sélection de souches bactériennes dotées de propriétés anti-inflammatoires, sur la base de leur capacité à diminuer l'expression du gène Rbxl.

## Description

La présente invention est relative à la sélection de souches bactériennes dotées de propriétés anti-inflammatoires.

Il a été rapporté que certaines bactéries lactiques possédaient des propriétés modulatrices de la réponse immunitaire, notamment l'amélioration de l'immunité vis-à-vis d'agents anti-infectieux, ou celle de la réponse anti-inflammatoire (cf. par exemple MAJAMAA et al., J. Pediatr. Gastroenterol. Nutr., 20, 333-339, 1995 ; GUERIN-DANAN et al., J. Nutr., 131, 111-117.2001 ; SHOMIKOVA et al., J. Pediatr. Gastroenterol. Nutr. 24, 399-404, 1997 ; LEWIS et al., Aliment. Pharmacol. Ther. 12, 807-822, 1998 ; ERICKSON et al., J. Nutr. 130, S403-409, 2000; ISOLAURI et al., Clin Exp Allergy 30, 1604-1610, 2000 ; MAJAMAA et al., J. Allergy. Clin. Immunol. 102, 179-185, 1997 ; CAMPIERI et al., Gastroenterology 116, 1246-1260, 1999; VANDERHOOF, J. Pediatr. Gastroenterol. Nutr. 30, S34-38, 2000 ; ISOLAURI et al., Am. J. Clin. Nutr. 73, S444-450, 2001 ; ISOLAURI, Gut, 52, 436-437, 2003).

Cependant, il apparaît que les effets induits par ces bactéries peuvent varier selon l'espèce et même, à l'intérieur d'une même espèce, selon la souche bactérienne utilisée. Il apparaît donc souhaitable de disposer de moyens de criblage permettant de sélectionner les bactéries possédant les propriétés recherchées.

Parmi les bactéries lactiques connues pour posséder des propriétés immunomodulatrices, et notamment anti-inflammatoires, on citera la souche de *Lactobacillus casei* DN 114001. Cette souche est décrite dans la Demande PCT WO 96/20607 au nom de : COMPAGNIE GERVAIS DANONE, et a été déposée le 30 décembre 1994, auprès de la CNCM (Collection Nationale de Cultures de Microorganismes), 25 rue du Docteur Roux, à Paris, sous le numéro I-1518. Il est montré, dans la Demande PCT WO 96/20607 que cette souche de *L. casei* est capable de réguler la réponse inflammatoire des entérocytes, et en particulier d'inhiber une réponse inflammatoire pathogène. Cette inhibition se traduit par une diminution de la production de NO et de celle de TNF par des cultures d'entérocytes, hyperactivés par l'addition de cytokines inflammatoires et de LPS pour reproduire les conditions d'un état inflammatoire pathogène.

Les inventeurs ont étudié l'effet de la souche CNCM I-1518 de *L. casei* sur la réponse inflammatoire des cellules épithéliales à *Shigella flexneri,* qui se traduit par l'induction de gènes codant pour des molécules pro-inflammatoires, parmi lesquelles, notamment, les chémokines CXCL1, CXCL2, et CCL20, et l'intégrine CD54. Ils ont constaté qu'une pré-exposition des cellules épithéliales à *L. casei* CNCM I-1518, avant l'infection par *Shigella,* diminue le niveau d'expression de ces molécules pro-inflammatoires, qui retourne à un niveau identique à celui observé chez des cellules épithéliales n'ayant pas été infectées. Ceci confirme donc les propriétés anti-inflammatoires de cette souche de *L. casei.*

Il est connu que l'expression des voies de signalisation pro-inflammatoires induites lors de l'infection par *S. flexneri* est sous la dépendance du facteur de transcription NF-κB (PHILPOTT et al., J Immunol 165, 903-914, 2000 ; GIRARDIN et al. EMBO Rep. 2, 736-742, 2001). Les Inventeurs ont recherché si *L. casei* CNCM I-1518 avait un effet sur l'expression ou l'activation de ce facteur de transcription. Ils ont observé qu'une préincubation des cellules épithéliales avec *L*. *casei* diminuait de 50% l'activation de NF-κB induite par *Shigella* ou par TNFα, et que cette diminution était due à une augmentation de la quantité de IκBα, l'inhibiteur de NF-κB.

IκBα inhibe l'activité de NF-κB en s'associant avec lui et en le retenant vers le cytoplasme bloquant ainsi sa translocation du cytoplasme vers le noyau. La dégradation de IκBα, qui s'effectue dans le protéasome, entraîne la libération de NF-κB, et permet sa translocation vers le noyau. Cette dégradation de IκBα nécessite sa phosphorylation par un complexe kinase (IBkinase), suivie de son ubiquitinylation par un complexe multiprotéique, dénommé E3-SCF^{β-TrCP}.

Les Inventeurs ont cherché à déterminer si l'augmentation de la quantité de IκBα était due à une augmentation de l'expression du gène correspondant, ou à une diminution de sa dégradation. Ils ont effectué une analyse du transcriptome d'entérocytes, après incubation avec *L. casei ;* ils n'ont observé aucune augmentation du niveau de transcription de IκBα. Parallèlement, ils ont recherché les différentes formes de IκBα : ils ont observé une forme phosphorylée (qui n'est en général pas détectable car rapidement ubiquitinylée et dégradée par le protéasome), mais n'ont pas observé de forme polyubiquitinylée. Ces résultats suggèrent que l'augmentation de IκBα résulte, au moins en partie, d'un blocage de l'étape d'ubiquitinylation.

En outre, dans le cadre de l'analyse du transcriptome des entérocytes après incubation avec *L. casei,* les Inventeurs ont observé une importante inhibition de la transcription du gène Ring box 1 *(rbx-1* /*rocl* /*hrtl).* Ce gène est connu pour être impliqué dans l'ubiquitinylation et le protéasome : il intervient au niveau de l'ubiquitine ligase E3 et a un rôle central dans la régulation de la dégradation de certaines protéines comme la béta-caténine. Ce gène est également connu pour intervenir au niveau de l'ubiquitinylation de IκBα (Tan P., Fuchs S.Y., Chen A. et al. 1999. Mol. Cell., 3 : 527-533).

Cette diminution de l'expression de *rbx-1* induite par *L. casei* peut donc expliquer l'absence de forme ubiquitinylée de IκBα et ainsi la non-dégradation de IκBα et donc la moindre activation de NF-κB.

La mise en évidence, par les Inventeurs, de la corrélation entre la capacité d'une bactérie à inhiber la transcription du gène Ring box 1 et sa capacité à inhiber la réponse inflammatoire médiée par le NF-κB, induite notamment par *Shigella* ou par TNFα, permet de proposer une nouvelle méthode de criblage de souches bactériennes dotées de propriétés anti-inflammatoires.

La présente invention a pour objet un procédé de criblage de bactéries dotées de propriétés anti-inflammatoires, caractérisé en ce qu'il comprend la sélection des bactéries capables de diminuer l'expression de *rbx-1* dans des cellules épithéliales en culture.

Généralement, un procédé conforme à l'invention comprend les étapes suivantes :
- l'incubation d'une culture de cellules épithéliales en présence de la bactérie à tester ;
- la détermination du niveau d'expression de *rbx-1* dans lesdites cellules épithéliales ;
- la comparaison du niveau d'expression observé en présence de la bactérie avec celui observé dans une culture témoin des mêmes cellules épithéliales incubées dans les mêmes conditions en l'absence de la bactérie à tester.

De préférence, l'incubation des cellules épithéliales est effectuée pendant au moins 16h avant la détermination du niveau d'expression de *rbx-1.* Avantageusement, les cellules épithéliales sont incubées en présence de 10 à 10³, de préférence en présence d'environ 10² u.f.c. bactériennes par cellule épithéliale.

Les bactéries utilisées sont issues, de préférence, de cultures en phase exponentielle .

Les cultures de cellules épithéliales sont utilisées de préférence à semi-confluence, car cette condition permet une meilleure invasion des cellules par *S*. *flexneri.*

Il est toutefois également possible d'utiliser des cellules Caco-2 confluentes, différenciées, obtenues après deux semaines de culture à confluence en changeant le milieu tous les deux jours.

Avantageusement, le niveau d'expression de *rbx-1* est déterminé en évaluant la quantité de transcrits de ce gène.

Différentes techniques permettant d'évaluer le niveau de transcription d'un gène déterminé sont connues en elles-mêmes ; à titre d'exemples non-limitatifs, on citera les techniques basées sur le transfert de Northern, les techniques basées sur l'utilisation de puces à ADN, ainsi que les techniques de RT-PCR quantitative, et notamment de RT-PCR en temps réel et les techniques de RT-PCR semi quantitative.

La détermination du niveau d'expression de *rbx-1* peut être également effectuée, par exemple, par dosage de la protéine Rbx-1 exprimée par les cellules épithéliales, ou à l'aide d'un anticorps dirigé contre cette protéine.

Les bactéries capables de diminuer le niveau d'expression de *rbx-1* sont sélectionnées à l'issue des différentes étapes mentionnées ci-dessus. Avantageusement, on sélectionne les bactéries en présence desquelles on observe une expression de *rbx-1* au moins 2 fois, de préférence au moins 4 fois inférieure à celle observée dans la culture témoin.

Le procédé de criblage conforme à l'invention peut *a priori* être mis en oeuvre sur toutes les espèces de bactéries. Il est cependant particulièrement avantageux de l'utiliser sur des espèces bactériennes employées dans l'industrie alimentaire, notamment sur des bactéries lactiques, telles que les lactobacilles, les lactocoques, les streptocoques thermophiles, ainsi que sur les bifidobactéries.

Les cellules épithéliales utilisées sont de préférence des cellules épithéliales humaines. A titre d'exemple, on citera les lignées Caco-2, HEK293, HeLa, HT-29, T-84, HCT-15,

L'invention peut en outre également concerner un ensemble de moyens (par exemple trousse de dosage ou plateforme technologique) pour le criblage de bactéries dotées de propriétés anti-inflammatoires comprenant :
- des cellules épithéliales sur lesquelles le test de criblage sera effectué ;
- des réactifs permettant de déterminer le niveau d'expression de *rbx1* dans ces cellules ;
- des bactéries à tester.

L'invention a aussi comme objet une bactérie susceptible d'être obtenue par le procédé selon l'invention (sauf L. casei CNCM I-1518).

L'invention a aussi comme objet l'utilisation d'une ou plusieurs bactéries (sauf *L. casei* CNCM I-1518) parmi celles obtenues par le procédé de criblage décrit ci dessus pour la fabrication d'une composition alimentaire ou pharmaceutique dotée de propriétés anti inflammatoires.

L'invention concerne enfin des compositions alimentaires comprenant une ou plusieurs bactéries (sauf *L. casei* CNCM I-1518) obtenues par le procédé de criblage décrit ci dessus.

La présente invention sera mieux comprise à l'aide du complément de description qui va suivre, qui se réfère à des exemples illustrant l'induction par *L. casei* d'une inhibition de la voie pro-inflammatoire induite par NF-κB, corrélée à une diminution de l'expression de *rbx-1.*

### EXEMPLE 1 : REDUCTION PAR L. CASEI DE LA PRODUCTION DE MOLECULES PRO-INFLAMMATOIRES INDUITE PAR S. FLEXNERI DANS DES CELLULES CACO-2

L'effet de *L. casei* sur l'expression de gènes codant pour les protéines pro-inflammatoires CXCL1, CXCL-2, Amphireguline, et ICAM-1, qui sont activés lors de l'infection par *Shigella,* a été étudié.

### Matériel et Méthodes :

### Culture des cellules Caco-2 :

Des cellules Caco-2 confluentes différenciées ont été cultivées dans des plaques de culture cellulaire à 6 puits. Dans ces conditions il y a 5 10⁵ cellules par puits. Le milieu de culture est du DMEM (InVitrogen), complémenté avec 10% de sérum de veau foetal décomplémenté, 100 U/ml de pénicilline, 100 µg/ml de streptomycine, et 1% d'acides aminés non-essentiels. Le milieu de culture est changé tous les 2 jours. Les cellules sont cultivées à 37°C, 10% CO₂.

### Culture de L. casei :

Les cultures sont effectuées à partir de colonies de *L. casei* (CNCM I-1518) préalablement cultivées sur milieu MRS-agar (DIFCO-BBL). Une colonie prélevée à l'anse de platine est utilisée pour ensemencer 12 ml de milieu MRS liquide (DIFCO-BBL) dans un tube FALCON de 15 ml. La culture est effectuée pendant une nuit à 37°C en tube fermé et sans agitation.

### Culture de S. flexneri (souche virulente M90T)

La souche M90T est une souche de référence (Sansonetti P. et al. (1982) Infect. Immun. 35, 852-860). Cette souche est cultivée sur boite d'agar contenant du rouge Congo afin d'isoler des colonies invasives. Une colonie est utilisée pour ensemencer 7 ml de milieu TSB (DIFCO-BBL) et est cultivée pendant une nuit à 37°C sous agitation. Cette préculture est diluée au 1/100 dans du milieu TSB frais, et incubée pendant 2 heures à 37°C sous agitation pour obtenir une culture en phase exponentielle (DO 600nm: 0,4).

### Incubation des cellules Caco-2 en présence de L . casei

Les plaques de culture contenant les cellules Caco-2 sont lavées en DMEM sans sérum (3 fois), puis incubées pendant 16 heures à 37°C, 5% CO₂ avec une suspension de *L. casei,* à raison de 100 bactéries/cellule, ou avec du milieu MRS.

### Infection des cellules Caco-2 par Shigella flexneri

Les plaques contenant les cellules Caco-2 sont lavées en DMEM sans sérum (3 fois), puis incubées pendant 3 heures à 37°C, 5% CO₂ avec une suspension de *S. flexneri* dans du DMEM sans sérum, à raison de 100 bactéries/cellule.

### Quantification par RT-PCR semi-quantitative

Les cellules Caco-2 sont lavées 3 fois par 3 ml de PBS à 4°C, puis lysées par 0.6 ml de tampon RLT contenant du 2-Mercapto-éthanol (1% final). L'ARN est extrait en utilisant le kit RNEasy, conformément au protocole préconisé par le fabricant (QIAGEN). La qualité et la quantité de l'ARN extrait sont respectivement déterminées par électrophorèse sur gel d'agarose 1 % contenant du bromure d'éthidium, et par spectrométrie (D.O. à 260 et 280 nm).

L'ADNc est préparé à partir de 5 microgrammes de l'ARN extrait, en utilisant la transcriptase inverse SuperScript II (InVitrogen) et des amorces oligo(dT) (PROMEGA).

Pour chaque réaction de PCR on utilise 5 microlitres de la préparation d'ADNc, diluée au 1/20, et une unité de polymérase EUROBIOTAQ (EUROBIO, Les Ulis, France), dans un volume réactionnel final de 50 microlitres.

Les amorces utilisées pour la PCR sont indiquées dans le tableau I ci-dessous :

**TABLEAU 1**

| Gène | Sens | Antisens |
|---|---|---|
| GAPDH | TGAAGGTCGGAGTCAACGGATTTGGT | CATGTGGGCCATGAGGTCCACCAC |
| Amphireguline | CTAGTAGTGAACCGTCCTCG | CTCCTTCATATTTCCTGACG |
| CXCLI | TGTCAACCCCAAGTTAGTTC | TCAATAATTAAGCCCCTTTG |
| CXCL2 | CCAAAGTGTGAAGGTGAAGT | ATGGGAGAGTGTGCAAGTAG |
| ICAM-1 | TGTCTAAAGGATGGCACTTT | ATGGCATATGTCTTCCACTC |

Après 35 cycles de PCR (45 secondes à 94°C, 45 secondes à 55°C, et 90 secondes à 72°C), suivis par une élongation pendant 7 minutes à 72°C, les produits d'amplification sont analysés par électrophorèse dans un gel d'agarose à 2% contenant du bromure. Les bandes correspondant aux produits de PCR sont quantifiées à l'aide du logiciel ImageQuant (AMERSHAM BIOSCIENCES).

### Résultats :

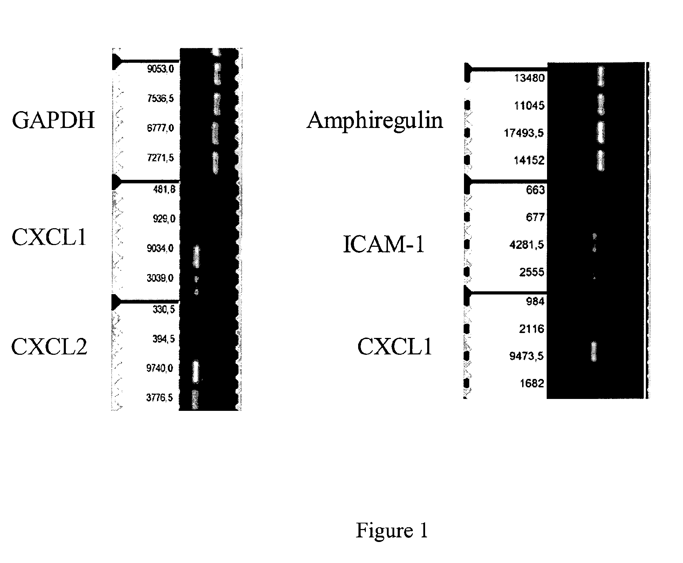

Les résultats obtenus sont représentés sur la Figure 1.

Légende de la figure 1 :
Pour chaque gène les pistes correspondant aux 4 conditions testées sont présentées de haut en bas :
   - cellules non infectées
   - cellules incubées avec *L. casei*
   - cellules infectées par *S*. *flexneri*
   - cellules incubées avec *L. casei* puis infectées avec *S*. *flexneri.*

Les résultats de la quantification des produits de PCR sont représentés par les chiffres figurant à coté de chaque piste.

La GAPDH est un gène domestique, utilisé à titre de contrôle pour normaliser la quantité d'ADN par réaction.

Ces résultats montrent que l'incubation des cellules Caco-2 en présence de *L. casei* entraîne une diminution de l'activation par *Shigella* des gènes d'inflammation CXCL1, CXCL-2, Amphireguline, et ICAM-1.

### EXEMPLE 2 : REDUCTION PAR L. CASEI DE L'ACTIVATION DE NF-κB INDUITE PAR S. FLEXNERI

Il est connu que la transcription des gènes pro-inflammatoires étudiés à l'Exemple 1 ci-dessus est sous le contrôle de NF-κB. En conséquence, l'effet de *L. casei* sur l'activation de NF-κB a été recherché, en étudiant l'expression, dans des cellules HEK293, du gène rapporteur de la luciférase, placé sous contrôle d'un promoteur inductible par NF-κB, selon le protocole précédemment décrit par GIRARDIN et al., (Science, 300, 1584-7, 2003)*.*

### Matériel et Méthodes :

### Culture des cellules:

Les cellules HEK293 sont cultivées dans des plaques de culture cellulaire à 24 puits, dans les mêmes conditions que celles décrites à l'Exemple 1 pour les cellules Caco-2.

Pour l'étude de l'activation de NF-κB, les cellules (10⁵ cellules/puits) sont transfectées avec un mélange de 75 ng du vecteur rapporteur Igκ-luciferase et de 400 ng du vecteur vide pcDNA3 (Invitrogen) par puits. Ce vecteur vide permet de contrôler que la transfection a bien eu lieu.

### Cultures bactériennes :

*L. casei* et *S*. *flexneri* sont respectivement cultivées comme décrit à l'exemple 1 ci-dessus.

### Incubation des cellules en présence de L.casei

L'incubation avec une suspension de *L. casei* ou du milieu MRS seul est effectuée comme décrit à l'Exemple 1. Pour les cellules transfectées avec le vecteur rapporteur, la suspension de *L. casei* ou le milieu MRS sont ajoutés 20 heures après transfection.

### Infection des cellules HEK par Shigella flexneri

L'infection par *S*. *flexneri* est effectuée comme décrit à l'Exemple 1. Les cellules sont incubées pendant 4 heures avec la suspension de *S. flexneri*

### Lyse des cellules

Les cellules sont lysées dans 100 microlitres d'un tampon (Tris pH :7,9 25mM, MgC12 8 mM, Triton 1 %, Glycérol 15 %) dans lequel on ajoute lmM DTT.

### Mesure de l'activation de NF-κB

Pour mesurer l'activité luciférase 10 microlitres de lysat cellulaire sont ajoutés à du tampon de lecture (Tris pH :7,9 25 mM, MgCl₂ 8 mM, Triton 1 %, Glycérol 15 %), additionné de 1mM DTT, 100mM ATP et Luciferin/K₂HPO₄- pH 7,58 2µM. La mesure est effectuée à l'aide d'un luminomètre (BECKMAN COULTER).

### Résultats :

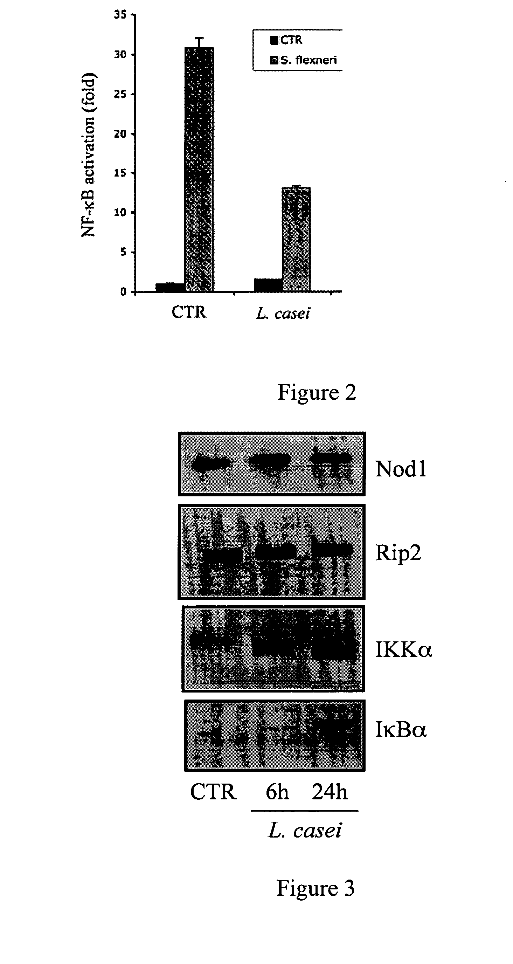

Les résultats obtenus sont illustrés par la Figure 2 (CTR= cellules contrôle, incubées en présence de MRS seul).

Ces résultats montrent que l'incubation des cellules HEK293 pendant 24 heures en présence de *L. casei* suffit à réduire d'environ 50% l'activation de la voie NF-κB induite par *S. flexneri.*

### EXEMPLE 3 : EFFET DE L. CASEI SUR L'EXPRESSION DE MOLECULES DE LA VOIE DE SIGNALISATION NOD1

Dans les cellules épithéliales, la voie de signalisation conduisant à l'activation de NF-κB suite à l'infection par *S. flexneri* implique la molécule Nod1 et une cascade de signalisation impliquant les molécules Rip2/RICK/Cardiak, le complexe IKK, et la protéine I-κBα. Il a été recherché si *L. casei* agissait au niveau de cette voie de signalisation, en étudiant son effet sur l'expression des protéines Nodl, Rip2, IKKα et I-κBα, évaluée par transfert de Western.

### Matériel et Méthodes :

Des cellules HEK293 ont été incubées en présence de *L. casei,* ou de milieu MRS, comme décrit à l'Exemple 1 ci-dessus, pendant 6 heures ou 24 heures, puis lysées par addition de 200 µl de tampon de Laemmli.

Après chauffage 5 minutes à 90°C, 10 µl de lysat sont déposés sur un gel SDS-PAGE à 15% d'acrylamide. Après migration, les protéines sont transférées sur membrane de nitrocellulose.

Les anticorps suivants ont été utilisés : α-Nod1 : anticorps polyclonal anti-Nodl (GIRARDIN et al., 2003, précité) : dilution 1/1000 ; α-Rip2 : anticorps polyclonal anti-Rip2 (CAYMAN LABS Inc.) : dilution 1/1000 ; α-IKKα : anticorps polyclonal anti-IKKα (SANTA CRUZ BIOTECHNOLOGY): dilution 1/200; α-IκBα : anticorps polyclonal anti-IκBα (SANTA CRUZ BIOTECHNOLOGY): dilution 1/500.

Après saturation par du tampon PBS additionné de 5% de lait, la membrane est incubée pendant une nuit avec l'anticorps choisi, à la dilution indiquée ci-dessus. Après plusieurs lavages en PBS-Tween, la membrane est incubée avec des anticorps de chèvre anti- IgG de lapin, marqués à la peroxydase (dilution : 1/1000). Après lavage, la membrane est incubée pendant 5 minutes avec du réactif ECL+ (AMERSHAM).

### Résultats :

Les résultats obtenus sont représentés sur la figure 3 (CTR= cellules contrôle, incubées en présence de MRS seul).

Ces résultats montrent que parmi les protéines testées, la seule dont le traitement par *L. casei* affecte l'expression est IκBα. Un traitement des cellules HEK293 durant 24 heures en présence de *L. casei* induit une augmentation significative de la quantité de cette protéine.

### EXEMPLE 4 : EFFET INHIBITEUR DE L. CASEI SUR LA DEGRADATION DE IκBα INDUITE PAR S. FLEXNERI

### Matériel et Méthodes :

Les cellules HEK293 ont été incubées pendant 24 heures en présence de *L. casei,* ou de milieu MRS, puis infectées par *S. flexneri* pendant différents temps (10', 20', 30', 40'), et lysées comme décrit à l'exemple 3 ci-dessus.

La dégradation de IκBα suivant l'infection par *S*. *flexneri* a été évaluée par transfert de Western, comme décrit à l'exemple 3 ci-dessus.

### Résultats :

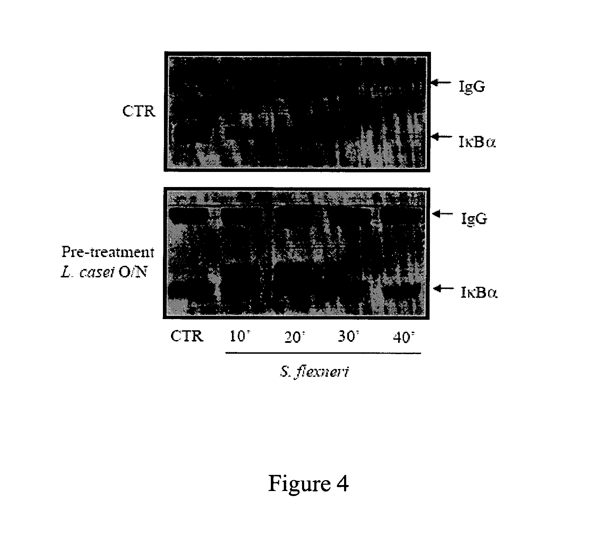

Les résultats obtenus sont représentés sur la figure 4.

Alors que *S. flexneri* induit une dégradation complète et très rapide de IκBα (en 30 minutes environ) dans les cellules contrôle (CTR) n'ayant pas subi de préincubation avec *L. casei* préalablement à l'infection,(Fig. 4, haut), le pré-traitement par *L. casei* inhibe cette dégradation (Fig. 4, bas). Les bandes « IgG » correspondent à la chaîne légère de l'anticorps polyclonal anti-IκBα, qui est utilisée à titre de contrôle interne.

### EXEMPLE 5 : EFFET INHIBITEUR DE L. CASEI SUR LES VOIES PRO-INFLAMMATOIRES INDUITES PAR LE TNFα

Il a été recherché si *L. casei* agissait également sur l'activation de NF-κB induite par le TNF-α, qui constitue une voie inflammatoire différente de celle induite par l'infection par *Shigella.*

### Matériel et Méthodes :

### Culture des cellules et incubation avec L. casei

Les cellules HEK293 ont été incubées comme décrit à l'exemple 2 ci-dessus, pendant 16 heures en présence de différentes concentrations (0,5, 2, ou 5% v/v) de culture de *L. casei* ou de milieu MRS. Dans le cas des cultures de *L. casei,* les concentrations utilisées correspondent respectivement à 10, 40, ou 100 bactéries/cellule.

### Stimulation par TNFα.

Les cellules sont lavées en DMEM sans sérum (3 fois), puis incubées pendant 4 heures à 37°C, 5% CO₂ en présence de 100ng/ml de TNFα.

### Mesure de l'activation de NF-κB :

L'activation de NF-κB est évaluée comme décrit à l'exemple 2.

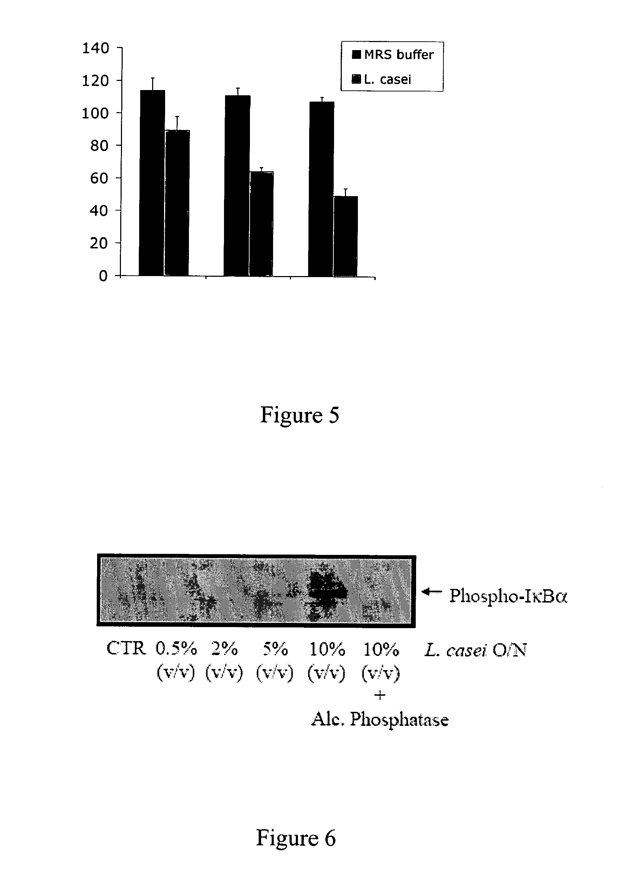

Les résultats obtenus sont représentés sur la figure 5.

Ces résultats montrent que l'addition de *L. casei* induit, même à faible dose (0,5%, soit 10 bactéries/cellule) une inhibition de l'activation de NF-κB. Cette inhibition apparaît dose dépendante, et atteint 50% lorsque *L. casei* est utilisée à raison de 10 bactéries/cellule.

*L. casei* exerce donc une activité anti-inflammatoire sur deux voies de signalisation pro-inflammatoires des cellules épithéliales : la voie Nod1 (infections bactériennes) et la voie TNFα.

### EXEMPLE 6: EFFET INHIBITEUR DE L. CASEI SUR L'ACTIVITE DU PROTEASOME

Les résultats présentés ici sur le niveau d'expression de IκBα peuvent être interprétés selon deux angles distincts. Soit cette augmentation d'expression témoigne d'une plus faible phosphorylation de IκBα (étape clé pour sa dégradation) et dans ce cas l'effet de *L. casei* doit être recherché en amont de la voie de signalisation, au niveau de IKKα ou de Rip2 ou encore de Nod1 ; en effet, ces molécules pourraient être activées plus faiblement sans que leur niveau d'expression en soit affecté. Ou encore, l'augmentation d'expression de IκBα résulterait d'une plus faible dégradation par le protéasome, à niveau de phosphorylation par la voie en amont équivalent. Le Western blot présenté à la figure 4 offre la possibilité d'envisager quelle interprétation est la plus pertinente. En effet, dans le cas de la culture des cellules en présence de *L. casei* avant infection, on remarque qu'une forme supplémentaire correspondant à IκBα est détectée migrant moins vite que la forme principale. Il est fort probable que cette forme corresponde à IκBα phosphorylée. Lorsque le protéasome est fonctionnel, cette forme phosphorylée est quasiment indétectable, tant le processus de dégradation de IκBα à la suite de sa phosphorylation est rapide.

Afin de vérifier cette hypothèse, la quantité de la forme phosphorylée de IκBα a été évaluée par transfert de Western en utilisant un anticorps reconnaissant spécifiquement cette forme.

### Matériel et Méthodes :

Les cellules HEK293 ont été incubées comme décrit à l'exemple 2, pendant 16 heures en présence de différentes concentrations (0,5, 2, 5, ou 10% v/v) de culture de *L. casei* ou de milieu MRS. Dans le cas des cultures de *L. casei,* les concentrations utilisées correspondent respectivement à 10, 40, 100, ou 200 bactéries/cellule.

Le transfert de Western a été effectué selon le protocole décrit dans l'exemple 3, en utilisant l'anticorps polyclonal anti-Phospho-IκBα (SANTA CRUZ BIOTECHNOLOGY) à la dilution 1/100.

Les résultats sont illustrés par la figure 6.

L'anticorps anti-Phospho-IκBα reconnaît une protéine dont la quantité augmente en fonction de la concentration en *L. casei.* Cette protéine n'est plus détectée après traitement par la phosphatase alcaline, ce qui montre qu'il s'agit d'une forme phosphorylée de IκBα. Ces résultats montrent que la culture des cellules HEK en présence de *L. casei* induit la stabilisation d'une forme phosphorylée de IκBα , et indiquent que c'est bien l'activité du protéasome, et non pas la voie de signalisation en amont aboutissant à la phosphorylation de IκBα, qui est affectée par *L. casei.*

### EXEMPLE 7 : EFFET INHIBITEUR DE L. CASEI SUR L'EXPRESSION DE RBX-1.

### Analyse du transcriutome :

### Matériel et Méthodes :

Les cellules Caco-2 semi-confluentes ont été incubées avec *L*. *casei* dans des boites de Pétri de 10 cm de diamètre. Le rapport bactéries/ cellules est de 100/1. Après 16 heures de culture à 37°C, 10% CO₂, on ajoute *S. flexneri* dans le même rapport bactéries/cellules. On centrifuge les boites de Petri 10 min à 1500 rpm, puis on incube pendant 3 heures à 37°C.

### -Extraction de l'ARN, et analyse sur puces d'ADN

Le protocole d'extraction des ARN est le même que celui décrit dans l'Exemple 1.

Le marquage est effectué selon le protocole d'AFFYMETRIX. Les puces utilisées sont les puces U133A. Après hybridation et révélation, les puces sont scannées avec un scanner AGILENT.

L'analyse des signaux d'hybridations, le calcul du bruit de fond, les analyses comparatives entre les différentes conditions sont réalisées avec le logiciel d'AFFYMETRIX MAS 5.

Le tableau II ci-après regroupe les principaux gènes intervenant dans l'activité du protéasome, et dont l'expression est diminuée après 24 heures d'incubation avec *L. casei.* Les chiffres représentent le rapport en échelle logarithmique (base 2) de l'expression des cellules incubées avec *L. casei vs* les cellules naïves.

**TABLEAU II**

| Diminution de l'expression | |
|---|---|
| Ring box 1 | -2,03 |
| Ubiquitin like 5 | -1,3 |
| Proteasome alpha 6 | -0,65 |

Un point très important à noter est la diminution d'expression du gène codant pour la protéine ring-box 1 (Rbx1). Cette protéine riche en cystéine joue un rôle pivot dans les processus d'ubiquitination et de dégradation de protéines impliquées dans l'inflammation (I-κB), dans la transcription (β-catenine), dans le cycle cellulaire (cdk-1) et dans la réponse à l'oxygène (HIF-1 alpha).

Afin de vérifier les résultats suggérés par l'analyse du transcriptome, l'effet de *L. casei* sur l'expression de *rbx-1* a été mesuré d'une part au niveau de la transcription du gène, par RT-PCR semi quantitative, et d'autre part au niveau de l'expression de la protéine Rbx-1, par transfert de Western.

### Mesure de l'expression de rbx-1 par RT-PCR semi quantitative.

Des cellules Caco-2 semi-confluentes sont incubées 16 heures avec *L*. *casei* (100 bactéries par cellule), comme décrit dans l'Exemple 1. L'extraction de l'ARN, la synthèse d'ADNc et la PCR sont également réalisées comme décrit dans l'Exemple 1, en utilisant pour la PCR, les amorces suivantes :

| Rbx1 | Sens | Antisens |
|---|---|---|
| | AAGAAGCGCTTTGAAGTGAA | GGTAACAGCAGGGAAAGTCA |

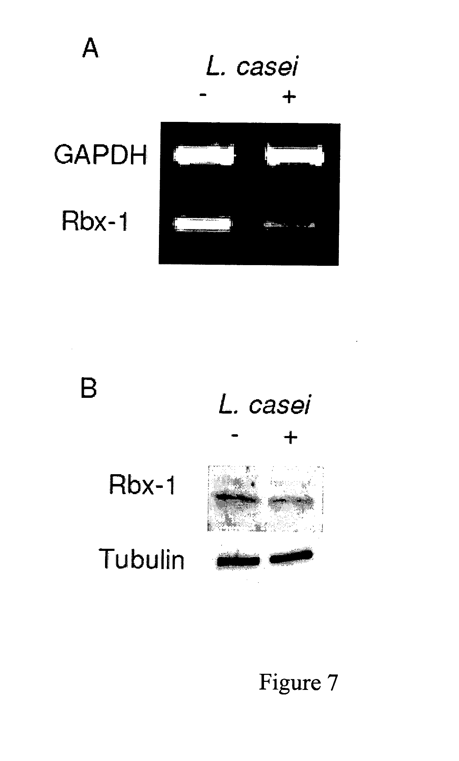

Les résultats sont illustrés par la figure 7A.

Ces résultats montrent que la transcription du gène *rbx-1* est fortement diminuée par l'incubation en présence de *L. casei.*

### Mesure de l'expression de la protéine Rbx-1 par transfert de Western

Des cellules Caco-2 semi-confluentes sont incubées 16 heures avec *L*. *casei* (100 bactéries par cellule), comme décrit dans l'Exemple 1.

Le transfert de Western a été effectué selon le protocole décrit dans l'exemple 3, en utilisant un anticorps polyclonal anti-Rbx-1 (SANTA CRUZ BIOTECHNOLOGY) à la dilution 1/500.

La Tubuline est utilisée comme contrôle interne.

Les résultats sont illustrés par la figure 7B.

Ces résultats montrent que la synthèse de la protéine Rbx-1 est diminuée lorsque les cellules sont incubées en présence de *L. casei,* et confirment donc les observations concernant la transcription du gène *rbx-1.*

## Revendications

1. Procédé de criblage de bactéries dotées de propriétés anti-inflammatoires, **caractérisé en ce qu'**il comprend :
- l'incubation d'une culture de cellules épithéliales en présence de la bactérie à tester ;
- la détermination du niveau d'expression de *rbx-1* dans lesdites cellules épithéliales ;
- la comparaison du niveau d'expression observé en présence de la bactérie avec celui observé dans une culture témoin des mêmes cellules épithéliales incubées dans les mêmes conditions en l'absence de la bactérie à tester ;
- la sélection de la bactérie en présence de laquelle le niveau d'expression observé est inférieur à celui observé dans la culture témoin.

2. Procédé selon la revendication 1, **caractérisé en ce que** l'incubation des cellules épithéliales en présence de la bactérie à tester est effectuée pendant au moins 16 heures avant la détermination du niveau d'expression de *rbx-1.*

3. Procédé selon une quelconque des revendications 1 ou 2, **caractérisé en ce que** les cellules épithéliales sont incubées en présence de 10 à 10³, u.f.c. bactériennes par cellule épithéliale.

4. Procédé selon une quelconque des revendications 1 à 3, **caractérisé en ce que** le niveau d'expression de *rbx-1* est déterminé en évaluant la quantité de transcrits de ce gène.

5. Procédé selon une quelconque des revendications 1 à 3, **caractérisé en ce que** le niveau d'expression de *rbx-1* est déterminé par dosage de la protéine Rbx-1 exprimée par les cellules épithéliales.

6. Procédé selon une quelconque des revendications 1 à 5, **caractérisé en ce que** l'on sélectionne une bactérie en présence de laquelle on observe une expression de *rbx-1* au moins 2 fois, de préférence au moins 4 fois inférieure à celle observée dans la culture témoin.

7. Procédé d'obtention d'une bactérie dotée de propriétés anti-inflammatoires, **caractérisé en ce qu'**il comprend :
- la mise en oeuvre d'un procédé de criblage selon une quelconque des revendications 1 à 6 ;
- la culture d'une bactérie sélectionnée par ledit procédé.

8. Bactérie susceptible d'être obtenue par un procédé selon la revendication 7, à l'exception de la souche de *L. casei* CNCM I-1518.

9. Bactérie **caractérisée en ce qu'**elle est capable d'induire une diminution de l'expression de *rbx-1* dans les cellules épithéliales, à l'exception de la souche de *L. casei* CNCM I-1518.

10. Utilisation d'une ou plusieurs bactéries selon l'une des revendications 8 ou 9, pour la fabrication d'une composition alimentaire ou pharmaceutique dotée de propriétés anti-inflammatoires.

11. Composition alimentaire comprenant une ou plusieurs bactéries selon les revendications 8 ou 9.

12. Ensemble de moyens pour le criblage de bactéries dotées de propriété anti-inflammatoires comprenant :
- des cellules épithéliales sur lesquelles le test de criblage sera effectué ;
- des réactifs permettant de déterminer le niveau d'expression de rbxl dans ces cellules ;
- des bactéries à tester.


SEQUENCE LISTING

<110>  COMPAGNIE GERVAIS DANONE
       INSTITUT PASTEUR
       PEDRON, Thierry
       GIRARDIN, Stéphane
       SANSONETTI, Philippe
       BOURDET-SICARD, Raphaëlle

<120>  Souches bactériennes induisant une diminution de l'expression de
       Rbx1

<130>  MJPah191/207

<160>  12

<170>  PatentIn version 3.3

<210>  1
<211>  26
<212>  DNA
<213>  Artificial

<220>
<223>  Amorce sens GAPDH

<400>  1
tgaaggtcgg agtcaacgga tttggt                                          26


<210>  2
<211>  24
<212>  DNA
<213>  Artificial

<220>
<223>  Amorce antisens GAPDH

<400>  2
catgtgggcc atgaggtcca ccac                                            24


<210>  3
<211>  20
<212>  DNA
<213>  Artificial

<220>
<223>  Amorce sens Amphireguline

<400>  3
ctagtagtga accgtcctcg                                                 20


<210>  4
<211>  20
<212>  DNA
<213>  Artificial

<220>
<223>  Amorce antisens Amphireguline

<400>  4
ctccttcata tttcctgacg                                                    20


<210>  5
<211>  20
<212>  DNA
<213>  Artificial

<220>
<223>  Amorce sens CXCL1

<400>  5
tgtcaacccc aagttagttc                                                    20


<210>  6
<211>  20
<212>  DNA
<213>  Artificial

<220>
<223>  Amorce antisens CXCL1

<400>  6
tcaataatta agcccctttg                                                    20


<210>  7
<211>  20
<212>  DNA
<213>  Artificial

<220>
<223>  Amorce sens CXCL2

<400>  7
ccaaagtgtg aaggtgaagt                                                    20


<210>  8
<211>  20
<212>  DNA
<213>  Artificial

<220>
<223>  Amorce antisens CXCL2

<400>  8
atgggagagt gtgcaagtag                                                    20


<210>  9
<211>  20
<212>  DNA
<213>  Artificial

<220>
<223>  Amorce sens ICAM-1

<400>  9
tgtctaaagg atggcacttt                                                    20


<210>  10
<211>  20
<212>  DNA
<213>  Artificial

<220>
<223>  Amorce antisens ICAM-1

<400>  10
atggcatatg tcttccactc                                                    20


<210>  11
<211>  20
<212>  DNA
<213>  Artificial

<220>
<223>  Amorce sens Rbx1

<400>  11
aagaagcgct ttgaagtgaa                                                    20


<210>  12
<211>  20
<212>  DNA
<213>  Artificial

<220>
<223>  Amorce antisens Rbx1

<400>  12
ggtaacagca gggaaagtca                                                    20